# EUROPEAN PATENT APPLICATION

(11) **EP 4 151 710 A1**
(43) Date of publication of application: **22.03.2023**
(21) Application number: 21198126.1
(22) Date of filing: 21.09.2021
(51) Int. Cl.: C12M 1/00, C12M 1/12

(54) **POLYMER MEMBRANE AND METHOD OF MANUFACTURING THE SAME**

(71) Applicant: Koninklijke Philips N.V., 5656 AG Eindhoven (NL)
(72) Inventor: BECKERS, Lucas Johannes Anna Maria, 5656 AE Eindhoven (NL); SANDERS, Renatus Hendricus Maria, 5656 AE Eindhoven (NL)
(74) Representative: Philips Intellectual Property & Standards

(57) **Abstract**

Provided is a method (100) of fabricating a polymer membrane (206) suitable for a cell culture device. The method comprises providing (102) a first polymer (200) having a first surface (201), and providing (104) a solution (204) on the first surface. The solution comprises a second polymer, a surfactant, and a solvent. The surfactant comprises surfactant molecules each having a polar moiety. The method further comprises evaporating (106) the solvent while the solution is on the first surface to provide the polymer membrane. The polymer membrane has a second surface (207) in contact with the first surface. At least the first surface of the first polymer has greater affinity for the polar moiety than the second polymer. Further provided is the polymer membrane per se, a fluidic device, such as a cell culture device, comprising such a polymer membrane, and a cell culturing method comprising encapsulating cells with one or more of the polymer membrane.

## Description

### FIELD OF THE INVENTION

This invention relates to a method of fabricating a polymer membrane, in particular a polymer membrane suitable for a cell culture device.

The invention also relates to a polymer membrane for a cell culture device.

The invention further relates to a cell culture device, such as for example a fluidic device, comprising such a polymer membrane.

The invention yet further provides a cell culturing method comprising encapsulating cells with one or more of such a polymer membrane.

### BACKGROUND OF THE INVENTION

*In vitro* testing of mammalian cells or tissue is an important technique to obtain clinically important information of the mammalian material under investigation. For example, biopsied mammalian cell or tissue material may be subjected to such testing in order to determine anomalies or disease in such mammalian material or to expose diseased mammalian material to drugs, e.g. experimental drugs, to monitor the response of the diseased mammalian material to such exposure. This approach, for example, is frequently used in oncological procedures. This can provide important insights in how a disease in an individual can be effectively treated without having to expose the individual to a range of potentially effective drugs, which can be undesirable for a number of reasons, such as drug toxicity. In addition, the efficacy of experimental drugs for existing diseases for which no established satisfactory drug treatment is yet available may be tested in this manner. There are many other well-known reasons for deploying such *in vitro* tests.

A common approach to such *in vitro* testing is to immobilize the mammalian material in a fluidic device, which is sometimes referred to as an organ-on-chip. In such an approach, the mammalian material is typically immobilized on a membrane separating two fluidic channels of the fluidic device, with a first channel being used to feed the mammalian material and the second channel being used to expose the mammalian material to a chemical compound or composition of interest such as a drug treatment, e.g. to test the efficacy and/or toxicity of the drug as previously explained. The fluidic device, or at least its membrane, may be made of an elastomer such that the fluidic device may be diced or sliced in order to obtain a slice of the membrane including the mammalian material for evaluation purposes, e.g. to evaluate the reaction of the mammalian material to the exposure to the chemical compound or composition of interest.

Cell samples, such as spheroids/organoids and biopsies, are often studied in well cell plates. Fluids are injected and taken away with pipets under visual inspection. The spheroids/organoids and biopsies are intended to stay in the wells. The use of fluidic devices where biopsies are placed on membranes is the latest state of the art practice. Fluidic handling protocols are currently in development. Fluids, e.g. containing cell nutrients and/or experimental drug(s), are passed over such membranes, and can risk sweeping spheroids/organoids away.

Another challenge in such *in vitro* testing is to ensure that the mammalian material, e.g. cells or tissue, are stabilized and develop normally on the membrane of the fluidic device such that they live for the duration of the test procedure. This is particularly challenging in oncology procedures where experiments can be rather lengthy. To this end, the mammalian material may be stabilized using a biocompatible material such as fibronectin, which ensures that the mammalian cells will keep multiplying by providing the cells with a chemical environment mimicking that of natural tissue.

Soft rubber or polymeric materials for the membrane are relatively apolar and hydrophobic, and do not generally have functional groups to adhere to or bond proteins. The implementation of soft rubber substrates or membranes in fluidic devices for cell culturing has generally been found to be difficult and expensive. Coatings or extra cellular matrices have also been found to be too easily flushed away in fluidic devices.

### SUMMARY OF THE INVENTION

The invention is defined by the claims.

According to examples in accordance with an aspect of the invention there is provided a method of fabricating a polymer membrane suitable for a cell culture device, the method comprising: providing a solution on a first surface of a first polymer, the solution comprising a second polymer, a surfactant, and a solvent, wherein the surfactant comprises surfactant molecules each having a polar moiety; and evaporating the solvent while the solution is on the first surface to provide said polymer membrane, the polymer membrane having a second surface in contact with the first surface, wherein at least said first surface of the first polymer has greater affinity for the polar moiety than the second polymer.

By at least the first surface, and in some examples the entirety, of the first polymer having greater affinity for the polar moiety than the second polymer, a preponderance of the surfactant molecules at the second surface of the polymer membrane may be orientated such that the polar moiety is made available at the second surface.

A higher affinity of the first polymer, or at least the first surface of the first polymer, relative to the second polymer for the polar moiety can be achieved by selecting a first polymer whose polarity at least at the first surface is higher than the second polymer. Alternatively or additionally, the first polymer may be selected to form stronger intermolecular interactions, such as hydrogen bonds, with the polar moiety at the first surface than the second polymer.

By the polar moiety of the surfactant molecules being made available at the second surface of the polymer membrane in this manner, the surface properties of the second surface may be particularly suitable for binding of, for example, a cell culturing protein, such as fibronectin, thereto.

Moreover, due to the first polymer, e.g. in the form of a foil, being employed for effecting the above-described orientation of the polar moiety, the polymer membrane can be relatively straightforwardly released therefrom, for instance by peeling the first polymer and the polymer membrane from each other, and/or by dissolving the first polymer in a solvent in which the polymer membrane is less soluble or insoluble so as to separate the polymer membrane from the first polymer. This provides a more convenient method of fabricating a polymer membrane having polar moieties available at a surface thereof.

In some embodiments, the second polymer comprises an elastomer. Alternatively or additionally, the second polymer has a Shore A hardness of 70 or less, more preferably 60 or less, and most preferably 20 or less. The deformation, e.g. resilient deformation, of such a second polymer can be particularly advantageous when the polymer membrane is employed in a cell culture device because the second polymer can be deformed, e.g. resiliently deformed, against a substrate and/or cover plate in order to seal fluid within the cell culture device.

Preferably, the second polymer comprises or consists of a polydiene, such as polybutadiene, polyisoprene or butyl rubber. Particular mention is made of polybutadiene due to its chemical and mechanical properties making it particularly suitable for employment in a cell culture device.

Moreover, in non-limiting examples in which the surfactant molecules, e.g. the hydrophobic moiety thereof, comprise an unsaturated carbon-carbon bond, the unsaturated carbon-carbon bonds included in the polybutadiene can provide sites at which the surfactant molecules can be grafted, for example covalently bonded, to the polybutadiene second polymer.

More generally, the method may further comprise crosslinking the second polymer and at least some of the surfactant molecules.

The second polymer may, for example, comprise an unsaturated carbon-carbon bond, e.g. a vinyl functional group, for example as in the case of polybutadiene, and/or a hydride functional group, and the at least some of the surfactant molecules each comprise an unsaturated carbon-carbon bond in the hydrophobic moiety, e.g. as in the case of the above-mentioned unsaturated surfactant, such as the unsaturated fatty acid. In such a non-limiting example, the crosslinking may be via a reaction between the unsaturated carbon-carbon bond of the surfactant and the unsaturated carbon-carbon bond, e.g. vinyl functional group, and/or hydride functional group of the second polymer.

More generally, the second polymer may comprise or consist of at least one selected from a polydiene, a polysiloxane, such as polydimethylsiloxane (PDMS), and a polyurethane.

A polysiloxane can, similarly to the above-described polydiene, have unsaturated carbon-carbon bonds (and/or hydride functional groups). This may enable the polysiloxane to crosslink with surfactant molecules comprising an unsaturated carbon-carbon bond.

It is noted that the lack of unsaturated carbon-carbon bonds in the case of, for example, a polyurethane second polymer may mean that this particular second polymer may not form crosslinks with surfactant molecules comprising an unsaturated carbon-carbon bond.

In some embodiments, the first polymer comprises or consists of one or more of polyvinylalcohol, polyurethane and polyethylene glycol. Such first polymers, e.g. in the form of a foil, can be releasable from the polymer membrane. Such release can be implemented in any suitable manner, noting, in particular, the properties of the first polymer.

For example, such release may be implemented by peeling and/or dissolving of the first polymer.

It is noted that such dissolving can be used, for instance, in the case of polyvinylalcolhol and polyethylene glycol, whereas the relatively low solubility of polyurethane may mean that peeling or stripping of a polyurethane first polymer from the polymer membrane is used instead of dissolving.

Moreover, such a polyvinylalcohol, polyurethane or polyethylene glycol first polymer may be more polar and/or form stronger intermolecular interactions, such as hydrogen bonds with, and thus have greater affinity for, the polar moiety than the second polymer, for example when the second polymer is a polydiene, such as polybutadiene.

In at least some embodiments, the solvent comprises or consists of one or more of a hydrocarbon solvent, such as heptane, an alcohol solvent, such as isopropylalcohol, and a ketone solvent, such as acetone. For example, the solvent comprises or consists of heptane, for example when the second polymer comprises or consists of a polydiene, such as polybutadiene.

The polar moiety may be a carboxylic acid group, an amine group or an amide group. Such functional groups can interact with, e.g. covalently bond to, a cell culturing protein.

In some embodiments, the surfactant comprises a fatty acid, with the carboxylic acid group of the fatty acid defining the polar moiety

In embodiments in which the surfactant comprises a fatty acid, the fatty acid may comprise or consist of an unsaturated fatty acid. The unsaturated carbon-carbon bond(s) of such a fatty acid may, for example, be used to crosslink the unsaturated fatty acid to the second polymer, e.g. via the above-described reaction involving the unsaturated carbon-carbon bond and/or hydride functional group included in the second polymer.

The unsaturated fatty acid may be one or more selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid and docosahexaenoic acid. Particular mention is made of linoleic acid, e.g. α-linolenic acid.

The method may comprise separating the polymer membrane and the first polymer. Such separating can be implemented in any suitable manner. For example, the separating can comprise delaminating, e.g. peeling, the first polymer from the polymer membrane and/or by dissolving the first polymer in a solvent in which the polymer membrane is less soluble or insoluble so as to separate the polymer membrane from the first polymer.

In at least some embodiments, the method comprises forming one or more apertures and/or grooves in the polymer membrane. Such groove(s) and/or aperture(s) may assist the polymer membrane to fulfil a function in, for instance, a cell culture device, such as enabling nutrients to be supplied and waste products to be removed from the cells via the groove(s) and/or aperture(s), and/or enabling monitoring of a cell sample.

The size/diameter of the apertures 210 can be selected according to what is most practical, e.g. for handling and/or microscopy. Such aperture(s) may, for example, have a diameter in the range of 1-1000 µm.

The forming may, for example, comprise laser drilling the polymer membrane.

In some embodiments, the method comprises functionalizing the second surface of the polymer membrane with a cell culturing protein, e.g. a human protein. The above-described polar moieties made available at the second surface facilitate this functionalization.

The cell culturing protein can, for example, comprise or consist of one or more of collagen, elastin, and fibronetin. In the case of fibronectin, this protein can be straightforwardly coupled to the polar moiety, particular in examples in which the polar moiety is a carboxylic acid group.

According to another aspect there is provided a polymer membrane obtainable from the method defined above.

According to still another aspect there is provided a fluidic device, such as a cell culturing device, comprising one or a plurality of the polymer membrane according to the present disclosure.

According to yet another aspect there is provided a cell culturing device , comprising: a polymer membrane and a further polymer membrane, the polymer membrane having a surface which opposes a further surface of the further polymer membrane, at least one of the polymer membranes comprising a surfactant comprising surfactant molecules each having a polar moiety at the respective surface or surfaces. The cell culturing device may comprise or consist of a fluidic device. A fluidic device being understood to comprise at least one channel for allowing or providing a fluid flow towards and/or away from the polymer membrane.

Encapsulating cells between the polymer membranes in this manner may lessen the risk of such cells, e.g. of a biopsy or spheroid/organoid, being flushed away when a fluid is provided to and/or withdrawn from the fluidic device.

One or both of the polymer membranes may, for example, be optically transparent, such as to enable optical analysis of the interior of the fluidic device between the polymer membranes. Such optical analysis can, for instance, be carried out using a suitable microscope.

A cell culturing protein may be bound to said one or both of the surfaces via the polar moiety, e.g. via covalent bonding of the cell culturing protein to the polar moiety.

One, or preferably both, of the polymer membranes may comprise one or more grooves and/or apertures. Such aperture(s) may, for example, have a diameter in the range of 1-1000 µm.

Aperture(s) in one or both of the membranes can, for instance, be optimized for a cell sample or type of cell sample, e.g. according to the dimensions of cells and/or cell layer(s) and/or spheroid(s)/organoid(s) and/or biopsy/ies.

In a non-limiting example, both polymer membranes are made of the same polymer, e.g. a polydiene, such as polybutadiene.

According to a further aspect there is provided a cell culturing method comprising immobilizing cells within a cell culturing device usingat least one of the polymer membrane according to the present disclosure. The immobilizing may comprise fixating and/or partially or entirely encapsulating the cells using the polymer membrane.

More generally, embodiments described herein in relation to the polymer membrane, fluidic device and cell culturing method may be applicable to the method of manufacturing the polymer membrane, and embodiments described herein in relation to the method of manufacturing the polymer membrane may be applicable to the polymer membrane itself, as well as the fluidic device and cell culturing method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIGs. 1A and 1B schematically depict a method of manufacturing a polymer membrane according to an example;
FIG. 2A schematically depicts orientating of surfactant molecules such as to make a polar group thereof available at a surface of the polymer membrane;
FIG. 2B schematically depicts polysiloxane curing and crosslinking with an unsaturated fatty acid surfactant taking place on a polymer surface;
FIG. 3 provides a plan view of an exemplary polymer membrane and a frame;
FIGs. 4A and 4B provide micrographs of part of an exemplary polymer membrane;
FIG. 5 provides a micrograph of another exemplary polymer membrane;
FIG. 6 schematically depicts encapsulation of a tissue sample by a plurality of polymer membranes;
FIG. 7 schematically depicts encapsulation of spheroids/organoids by a plurality of polymer membranes;
FIGs. 8A to 8C provide micrographs of an exemplary polymer membrane for cell sample encapsulation; and
FIG. 9 provides a micrograph of an exemplary linoleic acid-modified polysiloxane polymer membrane.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a method of fabricating a polymer membrane suitable for a cell culture device. The method comprises providing a first polymer having a first surface, and providing a solution on the first surface. The solution comprises a second polymer, a surfactant, and a solvent. The surfactant comprises surfactant molecules each having a polar moiety. The method further comprises evaporating the solvent while the solution is on the first surface to provide the polymer membrane. The polymer membrane has a second surface in contact with the first surface. At least the first surface of the first polymer has greater affinity for the polar moiety than the second polymer. Further provided is the polymer membrane per se, a fluidic device, such as a cell culture device, comprising such a polymer membrane, and a cell culturing method comprising encapsulating cells with one or more of the polymer membrane.

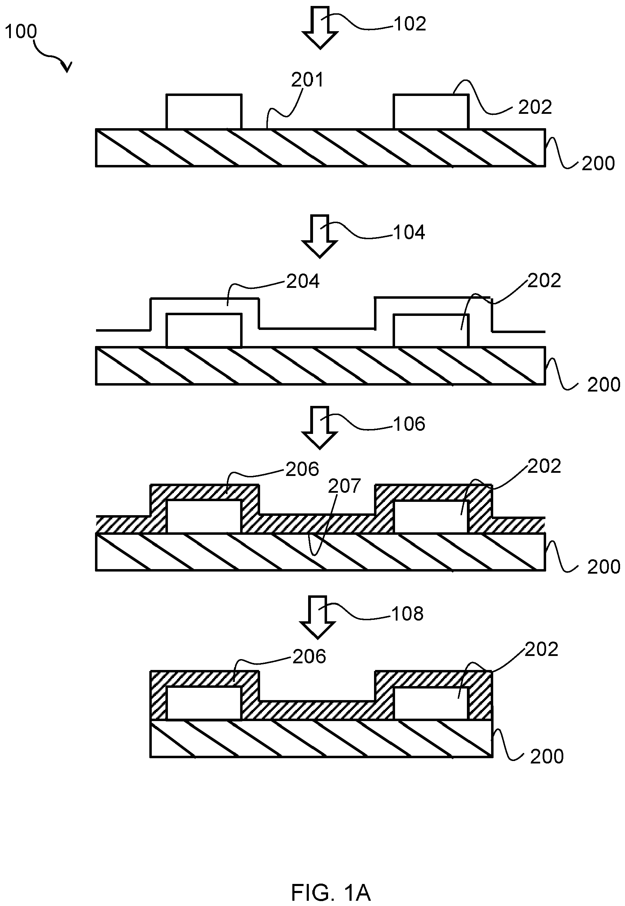

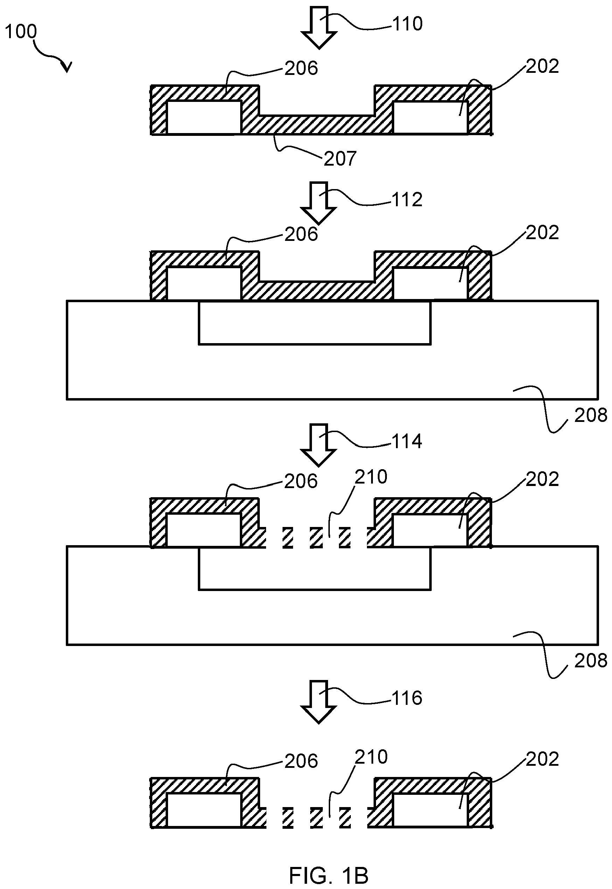

FIGs. 1A and 1B schematically depict a method 100 for fabricating a polymer membrane according to a non-limiting example. The method comprises providing 102 a first polymer 200 having a first surface 201.

In the non-limiting example shown in FIGs. 1A and 1B, a frame 202 is also provided on the first polymer 200, in particular on the first surface 201. The frame 202 can facilitate subsequent manufacturing steps, such as forming one or more grooves and/or apertures in the polymer membrane, as will be described in more detail herein below. In other examples, such a frame 202 can be introduced at a different stage of the method 100, or omitted altogether.

The method 100 comprises providing 104 a solution 204 on the first surface 201. The solution comprises a second polymer, a surfactant, and a solvent. The second polymer corresponds to the polymeric component of the polymer membrane.

The providing 104 the solution 204 can be implemented in any suitable manner, such as by dispensing, spin coating, etc. The manner in which the solution 204 is provided 104 can, for example, determine the thickness of the polymer membrane, as will be discussed in more detail herein below.

The surfactant comprises surfactant molecules each having a polar moiety. The surfactant can also include a hydrophobic moiety.

The polar moiety may provide a site to which, for example, a cell culturing protein, such as fibronectin, can bind, for example by grafting, e.g. covalent bonding, to the polymer membrane. Such binding of the cell culturing protein to the polymer membrane can, in at least some examples, be implemented in a later step of the method 100.

In some embodiments, the polar moiety is a carboxylic acid group, an amine group or an amide group. Such functional groups can interact with, e.g. covalently bond to, a cell culturing protein.

Non-limiting examples of an amine polar moiety-comprising surfactant are octadecanamide, also known as N-(3-dimethylaminopropyl)octadecamide, and polylysine, e.g. poly-D-lysine.

Preferably, the surfactant comprises, or is in the form of, a fatty acid, with the carboxylic acid group of the fatty acid defining the polar moiety.

More generally, in some embodiments the method 100 further comprises curing the second polymer and/or crosslinking the second polymer to at least some of the surfactant molecules.

Such curing can be implemented in any suitable manner. Curing may be implemented with UV radiation and/or thermally, in other words by heating. Curing/crosslinking the second polymer will be described in more detail herein below.

In some embodiments, the surfactant molecules, e.g. the hydrophobic moiety thereof, comprise an unsaturated carbon-carbon bond. Such an unsaturated carbon-carbon bond can, in some non-limiting examples, be crosslinked to the second polymer.

The above-mentioned fatty acid can, for instance, comprise or consist of an unsaturated fatty acid. The unsaturated carbon-carbon bond(s) in the hydrophobic tail of such an unsaturated fatty acid is or are crosslinkable to the second polymer.

Such an unsaturated fatty acid is, for instance, one or more selected from myristoleic acid, palmitoleic acid, sapienic acid, oleic acid, elaidic acid, vaccenic acid, linoleic acid, linoeladic acid, linoleic acid, e.g. α-linolenic acid, arachidonic acid, eicospaentaenoic acid, erucic acid and docosahexaenoic acid.

In the context of the present disclosure, where reference is made to an unsaturated fatty acid, it is to be understood that this is intended to refer to any organic compound comprising a carboxylic acid head group covalently bound to an aliphatic tail comprising 10-30 carbon atoms and at least one carbon-carbon double bond in the aliphatic tail. The aliphatic tail may terminate in a non-aromatic ring structure, e.g. a five-membered or 6-membered cyclopentyl or cyclohexyl group, which may itself contain at least one double bond. A non-limiting example of an organic compound intended to fall within the above definition of an unsaturated fatty acid is retinoic acid. Many other compounds in addition to the example compounds explicitly mentioned in the present disclosure will be immediately apparent to the skilled person.

The second polymer and the surfactant, e.g. fatty acid, such as an unsaturated fatty acid, may be in any suitable ratio. Preferably, the surfactant is present in the solution 204 and/or in the polymer membrane in a range of 0.05-35% by weight of the second polymer. This may assist the polymer membrane 206 to retain sufficient polymeric character, e.g. deformability.

The second polymer can be any suitable polymer suitable for use in a cell culture device. The second polymer can itself be a biocompatible polymer. Alternatively or additionally the surfactant can itself be biocompatible.

It is nonetheless noted that the second polymer and the surfactant need not themselves be biocompatible due to the above-mentioned subsequent functionalization of the polymer membrane with a cell culturing protein, such as fibronectin.

More generally, the second polymer may comprise or consist of an elastomer. Alternatively or additionally, the second polymer has a Shore A hardness of 70 or less, more preferably 60 or less, and most preferably 20 or less. The deformation, e.g. resilient deformation, of such a second polymer can be particularly advantageous when the polymer membrane is employed in a cell culture device because the second polymer can be deformed, e.g. resiliently deformed, against a substrate (not visible in FIGs. 1A and 1B) and/or cover plate in order to seal fluid within the cell culture device.

In some embodiments, the second polymer comprises or consists of at least one selected from a polydiene, a polysiloxane, such as polydimethylsiloxane (PDMS), and a polyurethane.

Preferably, the second polymer comprises or consists of a polydiene, such as polybutadiene, polyisoprene or butyl rubber. Particular mention is made of polybutadiene due to its chemical and mechanical properties making it particularly suitable for employment in a cell culture device.

Moreover, in non-limiting examples in which the surfactant molecules, e.g. the hydrophobic moiety thereof, comprise an unsaturated carbon-carbon bond, the unsaturated carbon-carbon bonds included in the polybutadiene can provide sites at which the surfactant molecules can be grafted, for example covalently bonded, to the polybutadiene second polymer.

It is noted that the lack of unsaturated carbon-carbon bonds in the case of, for example, a polyurethane second polymer may mean that this particular second polymer may not form crosslinks with surfactant molecules comprising an unsaturated carbon-carbon bond.

In some embodiments, the second polymer may comprise an unsaturated carbon-carbon bond, e.g. a vinyl functional group, for example as in the case of polybutadiene, and/or a hydride functional group, for example as in the case of a polysiloxane, and the at least some of the surfactant molecules each comprise an unsaturated carbon-carbon bond in the hydrophobic moiety, e.g. as in the case of the above-mentioned unsaturated surfactant, such as the unsaturated fatty acid. In such a non-limiting example, the crosslinking may be via a reaction between the unsaturated carbon-carbon bond of the surfactant and the unsaturated carbon-carbon bond, e.g. vinyl functional group, and/or hydride functional group of the second polymer.

The solvent can be any solvent in which the second polymer and the surfactant can be dissolved and/or dispersed in order to enable providing 104 of the solution 204 on the first surface 201, but which can also be evaporated in a subsequent step of the method 100. The solvent may also not or only minimally solvate the first surface 201 of the first polymer 200 on which the solution 204 is provided so that the polymer membrane can be formed on the first surface 201.

In at least some embodiments, the solvent comprises or consists of one or more of a hydrocarbon solvent, such as heptane, an alcohol solvent, such as isopropylalcohol, and a ketone solvent, such as acetone. For example, the solvent comprises or consists of heptane, for example when the second polymer comprises or consists of a polydiene, such as polybutadiene.

Where polybutadiene is used as, or included in, the second polymer, the polybutadiene may be made using any suitable polymerization process. Particularly preferred is a polybutadiene formed in a Nd or Li-catalyzed polymerization reaction, as it is well-known per se that such polybutadiene has a low degree of branching (typically below 5%) and a low degree of polydispersity (Mw/Mn) of around 2. Li-catalyzed polybutadiene is particularly preferred due to its high degree of 1,2-vinyl content (about 11%). However, other types of polybutadiene, e.g. polybutadiene obtained from a Co, Ni or Ti-catalyzed polymerization reaction may be used instead.

Where a polydiene such as polybutadiene is used as the second polymer, the above-described crosslinking reaction between the second polymer and the surfactant, e.g. unsaturated fatty acid, may be catalyzed using a peroxide catalyst (e.g. provided in addition to UV radiation and/or heating). Any suitable peroxide can be used to accelerate curing/crosslinking of the polydiene, e.g. polybutadiene, such as di-cumyl peroxide.

In another advantageous embodiment, a Li-catalyzed polybutadiene is used having a degree of branching in a range of 5-15% as it has been found that when the degree of branching of the polybutadiene is in this range, the reactivity of the polybutadiene with the unsaturated fatty acid can be improved. It is noted for the sake of completeness that it is well-known per se how to control the degree of branching when synthesizing polybutadiene such that this will not be further explained for the sake of brevity only.

Another class of second polymer that is specifically mentioned is silicones, in other words polysiloxanes. Such silicones may comprise a PDMS backbone including vinyl moieties to facilitate crosslinking through Pt-catalyzed addition reactions, e.g. with (poly methyl) hydrogen siloxanes. Instead of or in addition to such crosslinking with (poly methyl) hydrogen siloxanes, the vinyl-functionalized PDMS backbone may be crosslinked with the surfactant, e.g. a surfactant comprising or in the form of an unsaturated fatty acid.

Curing of the polysiloxane may, for example, be initiated after mixing two parts: part A comprising a siloxane and a platinum catalyst and part B comprising the crosslinker.

In some non-limiting examples, after adding and mixing the surfactant the curing reaction may be slowed down. There accordingly may be a delay/idle time in which to provide the solution 204 onto the first polymer 200, e.g. via dispensing, spin coating etc. By subsequently heating the solution 204, for example up to a temperature in the range of 80-120°C, the curing may accelerate, for instance such that the curing is complete in less than 1 hour.

More generally, the cured/crosslinked polysiloxane may be formed from a multi-part starting material as previously explained in order to prevent premature crosslinking of the polysiloxane. Such multi-part starting materials are well-known per se; for example, such multi-part starting material kits are marketed by Wacker Chemie AG from Munich, Germany under the tradename Elastosil.

Such silicones may be formed by a crosslinking reaction between a vinyl-functionalized linear or branched silicone monomer or oligomer, e.g. a T-branched or Q-branched silicone monomer or oligomer and a linear hydride-functionalized silicone monomer or oligomer, between a hydride-functionalized linear or branched silicone monomer or oligomer, e.g. a T-branched or Q-branched silicone monomer or oligomer and a linear vinyl-functionalized silicone monomer or oligomer or mixtures of vinyl-functionalized and hydride-functionalized linear or branched silicone monomers or oligomers, e.g. a T-branched or Q-branched silicone monomer or oligomer and/or a mixture of linear hydride-functionalized and vinyl-functionalized silicone monomer or oligomer.

For example, a two-component silicone may be formed by a crosslinking reaction between a linear component 1 and a linear component 2, which each may correspond to the general formula below:

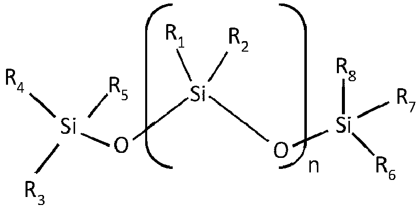

In component 1, R₁ and R₂ are individually selected from C₁-C₃ alkyl, R₃-R₈ are individually selected from C₁-C₃ alkyl and vinyl, with the proviso that at least one of R₃-R₅ and at least one of R₆-R₈ is vinyl. Preferably, at least three of R₃-R₈ are vinyl. In all embodiments, n may be in the range of 100 - 200,000. In a specific embodiment of component 1, each of the groups Ri-Rs that is an alkyl group is a methyl group, i.e. the component 1 is vinyl-functionalized PDMS having terminal vinyl groups.

In component 2, R₁ is hydrogen, R2 = C₁-C₃ alkyl and R₂-R₈ are individually selected from C₁-C₃ alkyl or hydrogen, with the proviso that at most one of R₃-R₅ and at most one of R₆₋₈ is hydrogen, and and n may have any suitable value, such as n = 3-1,000 or more specifically n = 3-10. In a specific embodiment of component 2, none of the groups R₃-R₈ are hydrogen. In another specific embodiment of component 2, each of R₂-R₈ are methyl.

Component 2 typically acts as a crosslinking agent for component 1. Such crosslinking reactions, which are typically Pt-catalyzed, are well-known per se, see for example WO 2009/147602 A2 and are therefore not explained in further detail for the sake of brevity.

Any suitable silicone elastomer may be used. It should be understood that when crosslinking such silicones in the presence of the surfactant, e.g. an unsaturated fatty acid, the covalent bond between the silicone and the surfactant alternatively may be formed by a reaction between a hydride functional group of the (crosslinked) silicone and the unsaturated fatty acid as suggested in the below reaction mechanism:

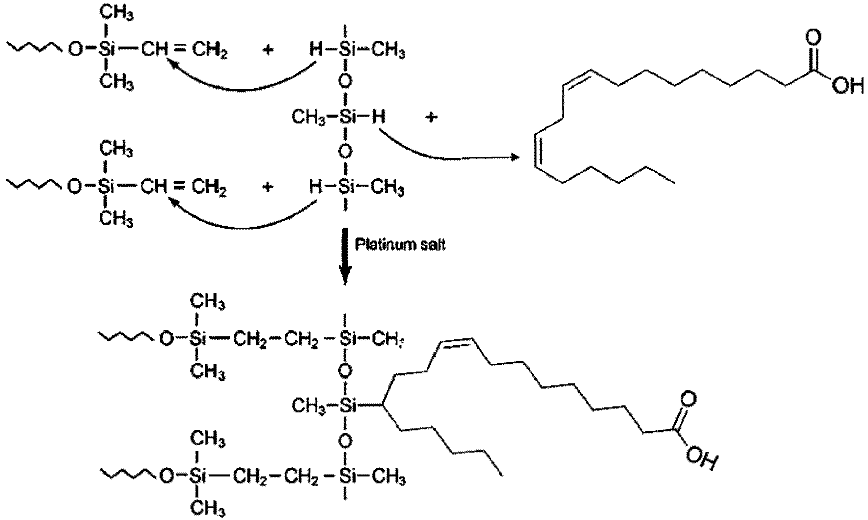

In order to prevent catalyst inhibition by the unsaturated fatty acid surfactant during such crosslinking reactions, the concentration of the unsaturated fatty acid may be limited, e.g. to a concentration of 1 wt.% or lower based on the solution 204. It has been found that below 1 wt.%, the fatty acid, e.g. linoleic acid, may not or only negligibly inhibit the crosslinking reactions, and curing and crosslinking at relatively high temperatures, e.g. higher than 80°C, can be achieved in spite of the presence of the fatty acid. Moreover, recipes based on such relatively low concentrations of the fatty acid, e.g. linoleic acid, have been found to work very well in terms of providing a polysiloxane-comprising or polysiloxane-based polymer membrane having polar moieties available at the surface of the polymer membrane.

In some non-limiting examples, a silicone backbone such as a (poly methyl) hydrogen siloxane backbone may be crosslinked with rubber-like polymers such as polybutadiene and polyisoprene, with the unsaturated fatty acid being incorporated in such a crosslinked product. This, for example, may be done to tune the water permeability of the final polymer membrane 206 as silicones typically have a rather open structure whilst such rubber-like polymers have a rather closed structure, such that the openness, i.e. the water permeability, of the crosslinked product can be tuned by the ratio of silicone and rubber-like polymer therein.

In this manner, the properties of the material according to this embodiment may be optimized for cell/protein interaction to build good scaffolds. For example, where the polymer membrane is a silicone-based, e.g. PDMS-based, a material that is highly permeable to water and other compounds (e.g. drugs) is obtained that facilitates maximized perfusion of these compounds into the cells on the polymer membrane. However, where real-time monitoring of drug consumption by cells is desirable, a polydiene-based material such as a polybutadiene-based material that is non-permeable to water and drugs may be preferable, as the rate of perfusion of these compounds to the cells can be controlled using, for instance, the density of apertures through the polymer membrane.

Returning to FIGs. 1A and 1B, the method 100 further comprises evaporating 106 the solvent while the solution 204 is on the first surface 201 to provide the polymer membrane 206. The thus formed polymer membrane 206 has a second surface 207 in contact with the first surface 201 of the first polymer 200, as schematically depicted in FIG. 1A.

The polymer membrane 206 can have any suitable thickness, depending, for example, on the manner in which the solution 204 is provided to the first surface 201 of the first polymer 200 and/or the intended function of the polymer membrane 206, e.g. in a fluidic device, such as a cell culture device.

In some embodiments, the polymer membrane 206 has a thickness in a range of 0.5-60 µm, such as 5-40 µm, for example 20-60 µm, e.g. about 40 µm.

The thickness of the polymer membrane 206 can be controlled, for instance, via the manner in which the solution 204 is provided to the first surface 201 of the first polymer 200. For example, dispensing the solution 204 onto the first surface 201 can enable fabrication of a polymer membrane 206 having a thickness of 10-60 µm, whilst spin coating the solution 204 on the first surface 201 can permit a polymer membrane 206 having a thickness of 0.5-10 µm to be made.

It is also noted that polymer membranes 206 suitable for, for instance, culturing spheroids, organoids or biopsies thereon have a thickness in the range of 20-60 µm, e.g. about 40 µm.

Limiting the thickness of the polymer membrane 206, e.g. to less than or equal to 60 µm may, in some examples, enable confocal microscopy to be used to monitor cell culture through the polymer membrane 206 in examples in which the polymer membrane 206 is employed in a cell culturing device.

More generally, at least the first surface 201 of the first polymer 200 has greater affinity for the polar moiety than the second polymer.

By at least the first surface 201, and in some examples the entirety, of the first polymer 200 having greater affinity for the polar moiety than the second polymer, a preponderance of the surfactant molecules at the second surface 207 of the polymer membrane 206 may be orientated such that the polar moiety is made available at the second surface 207.

A higher affinity of the first polymer 200, or at least the first surface 201 of the first polymer 200, relative to the second polymer for the polar moiety can be achieved by selecting a first polymer 200 whose polarity at least at the first surface 201 is higher than the second polymer. Alternatively or additionally, the first polymer 200 may be selected to form stronger intermolecular interactions, such as hydrogen bonds, with the polar moiety at the first surface 201 than the second polymer.

Thus, the higher polarity of the first polymer 200 (at least) at the first surface 201 relative to the polarity of the second polymer can mean that the surfactant molecules orientate such that the polar moiety interacts, e.g. via permanent dipole-permanent dipole-type interactions, with the first surface 201 of the first polymer 200.

In embodiments in which the surfactant molecules each comprise a hydrophobic moiety, e.g. a hydrophobic tail moiety, the lower polarity of the second polymer relative to (at least the first surface 201) of the first polymer 200 further means that the hydrophobic moiety, e.g. hydrophobic tail moiety, preferentially interacts with the second polymer, thereby to assist this orientation of the surfactant molecules to make the polar moiety available at the second surface 207 of the polymer membrane 206.

By the polar moiety of the surfactant molecules being made available at the second surface 207 of the polymer membrane 206 in this manner, the surface properties of the second surface 207 may be particularly suitable for binding of, for example, a cell culturing protein, such as fibronectin, thereto.

Such binding can, in at least some examples, comprise covalent bonding of the cell culturing protein to the polar moiety. In a non-limiting example, the polar moiety is a carboxylic acid group. In such an example, fibronectin can be covalently bonded to the polymer membrane via a reaction with the carboxylic acid group.

A well-documented binding protocol for covalently binding fibronectin to surface carboxylic acid groups using 1-ethyl-3-(3-dimethylamino) propyl carbodiimide hydrochloride (EDC) and N-hydroxysuccinimide (NHS) is given below:

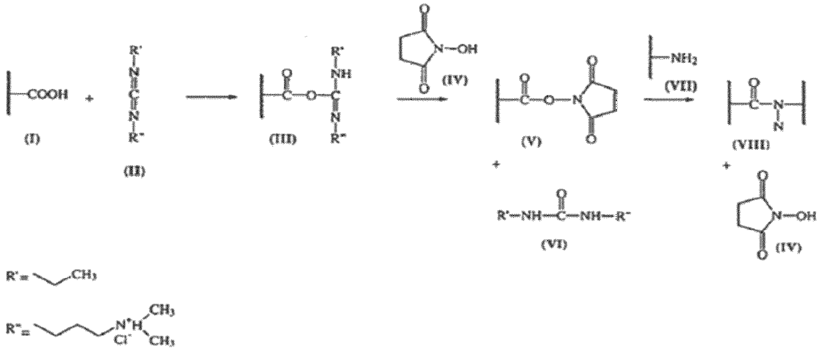

A description of this protocol can be found in: L.H.H.Olde Daminnk, P,J,Dijkstra, M.J.A.Luyn, P.B.v.Wachem, P.Nieuwenhuis, and J.Feijen, "Cross-linking of dermal sheep collagen using a water-soluble carbodiimide", Biomaterials, 17(8) pp. 765-774 (1996).

Moreover, due to the first polymer 200 being employed for effecting the above-described orientation of the polar moiety, the polymer membrane 206 can be relatively straightforwardly released therefrom, for instance by peeling the first polymer 200, e.g. first polymer foil, and the polymer membrane 206 from each other, and/or by dissolving the first polymer 200 in a solvent in which the polymer membrane 206 is less soluble or insoluble so as to separate the polymer membrane 206 from the first polymer 200. This provides a more convenient method 100 of fabricating a polymer membrane 206 having polar moieties available at a surface thereof.

In some embodiments, the first polymer 200 comprises or consists of one or more of polyvinylalcohol, polyurethane and polyethylene glycol. Such first polymers 200, e.g. in the form of a foil, can be releasable from the polymer membrane 206, e.g. by peeling and/or dissolving of the first polymer 200.

Particular mention is made of polyvinylalcohol and polyethylene glycol because the water-solubility of such first polymers 200 means that water can be used to remove the first polymer 200 from the polymer membrane 206.

It is noted that in the case of, for example, a polyurethane first polymer 200, the relatively low solubility of polyurethane may mean that peeling or stripping of the polyurethane first polymer 200 from the polymer membrane 206 is used instead of dissolving.

Moreover, such a polyvinylalcohol, polyurethane or polyethylene glycol first polymer 200 may be more polar and/or form stronger intermolecular interactions, such as hydrogen bonds with, and thus have greater affinity for, the polar moiety than the second polymer, for example when the second polymer is a polydiene, such as polybutadiene.

Employing the first polymer 200 may have further advantages, for example relating to manufacturability. Returning to FIGs. 1A and 1B, the method 100 may comprise cutting 108 a laminate comprising the polymer membrane 206 layered on the first polymer 200 to a desired shape and/or size. Such cutting 108 is enabled by it being a cuttable (first) polymer 200 which is employed to orientate the surfactant molecules such as to make the polar moieties available at the second surface 207 of the polymer membrane 206.

Referring to FIG. 1B, the method 100 may comprise separating 110 the polymer membrane 206 and the first polymer 200. Such separating 110 can be implemented in any suitable manner. For example, the separating 110 can comprise delaminating, e.g. peeling, the first polymer 200 from the polymer membrane 206 and/or by dissolving the first polymer 200 in a solvent in which the polymer membrane 206 is less soluble or insoluble so as to separate the polymer membrane 206 from the first polymer 200, as previously described.

In the non-limiting example shown in FIG. 1B, the frame 202 is also separated, e.g. delaminated, from the first polymer 200 together with the polymer membrane 206, while the frame 202 continues to hold the polymer membrane 206.

In some embodiments, the second polymer is optically transmissive, e.g. optically transparent, thereby to permit optical interrogation of, for instance, cells located on the other side of the polymer membrane 206 with respect to the apparatus, e.g. microscope, used for the optical interrogation.

In some embodiments, such as in the exemplary method 100 shown in FIGs. 1A and 1B, one or more grooves and/or apertures 210 are formed in the polymer membrane 206 in step 114. Such groove(s) and/or aperture(s) 210 may assist the polymer membrane 206 to fulfil a function in, for instance, a cell culture device, such as enabling nutrients to be supplied and waste products to be removed from the cells via the groove(s) and/or aperture(s) 210.

The size/diameter of the apertures 210 can be selected according to what is most practical, e.g. for handling and/or microscopy. Such aperture(s) may, for example, have a diameter in the range of 1-1000 µm.

For certain applications, such as when cell layers are desired to grow on the polymer membrane 206, the one or more aperture(s) 210 may be smaller than 8 µm in diameter to order to retain cells on the correct side of the polymer membrane 206, for instance to avoid or minimize cells crossing over, e.g. when epithelial cells are at one side of the polymer membrane 206 and endothelial cells are on the other side.

For spheroids and organoids, the aperture(s) 210 may have a diameter in the range of 50-250 µm, depending on the dimensions of the spheroids and organoids.

When the cell culture device includes a fluidic system, it may be particularly important for spheroids and organoids that these are not unintentionally washed away by the flow and that they remain at their positions. Hence, the diameter of the aperture(s) 210 may be selected to retain such spheroids or organoids, as the case may be, on the intended side of the polymer membrane 206.

For biopsies, the aperture(s) 210 may have a diameter in the range of 50-1000 µm. For example, biopsies can be 4 mm long, 0.5 mm wide, and 300 µm high.

For spheroids, organoids and biopsies the cell culture device may comprise a plurality of such polymer membranes 206, e.g. with a pair of such polymer membranes 206 opposing each other.

In a non-limiting example, which will be further explained herein below, cell culturing may take place on one of the polymer membranes 206, with another polymer membrane 206 functioning as a setter/keeper to allocate the spheroids, organoids or biopsy between the polymer membranes 206.

For monitoring growing, treatment and staining the spheroids, organoids and biopsies it may be important that they are fixed in position. Particularly for biopsies the history of geometry may be key because biopsies can have a tumor side and a healthy tissue side. Where treatments, such as chemotherapy treatments, are being evaluated in may be critical to observe how the tumor tissue and the healthy tissue respond.

In the non-limiting example shown in FIGs. 1A and 1B, such one or more grooves and/or apertures 210 are formed in the polymer membrane 206 via laser drilling, in other words laser drilling ablation process. In this particular example, the method comprises mounting 112 the polymer membrane 206 on a holder 208 to enable subsequent laser drilling of the grooves and/or apertures 210 in step 114.

Any suitable conditions can be employed for such laser drilling. The following is provided by way of a non-limiting example: wavelength: 1025 nm; pulse duration: 350 fs; average power: 500 mW; Spot size: 15∼20 µm; number of pulses: 25∼100.

The polymer membrane 206 can be mounted on the holder 208 via the frame 202, in other words while the polymer membrane 206 is attached to the frame 202, as shown.

Once the grooves and/or apertures 210 have been formed in the polymer membrane 206 via the above-described laser drilling process, the holder 208 may be removed in step 116.

In other non-limiting examples, an alternative technique for forming such grooves and/or holes 210 may be employed, such as for example by shaping the first surface 201 of the first polymer 200 such that such grooves and/or holes 210 are formed in the polymer membrane 206 following evaporation of the solvent.

In some embodiments, the method 100 further comprises functionalizing the second surface 207 of the polymer membrane 206 with a cell culturing protein. The polar moieties made available at the second surface 207 facilitate this functionalization, as previously described.

The cell culturing protein can comprise or consist of one or more of collagen, elastin, and fibronetin. In the case of fibronectin, this protein can be coupled, for example in the above-described manner using EDC and NHS, to a polar moiety in the form of a carboxylic acid group.

In non-limiting examples in which the first polymer 200 can be removed from the polymer membrane 206 by dissolving the first polymer 200, this process can be combined with protein coupling by including the protein and any coupling agents, such as EDC and NHS, in the same solvent used for dissolving the first polymer 200. For example, when water is used to remove, or assist in removing, a polyvinylalcohol first polymer 200, the protein coupling may be carried out using water as a solvent.

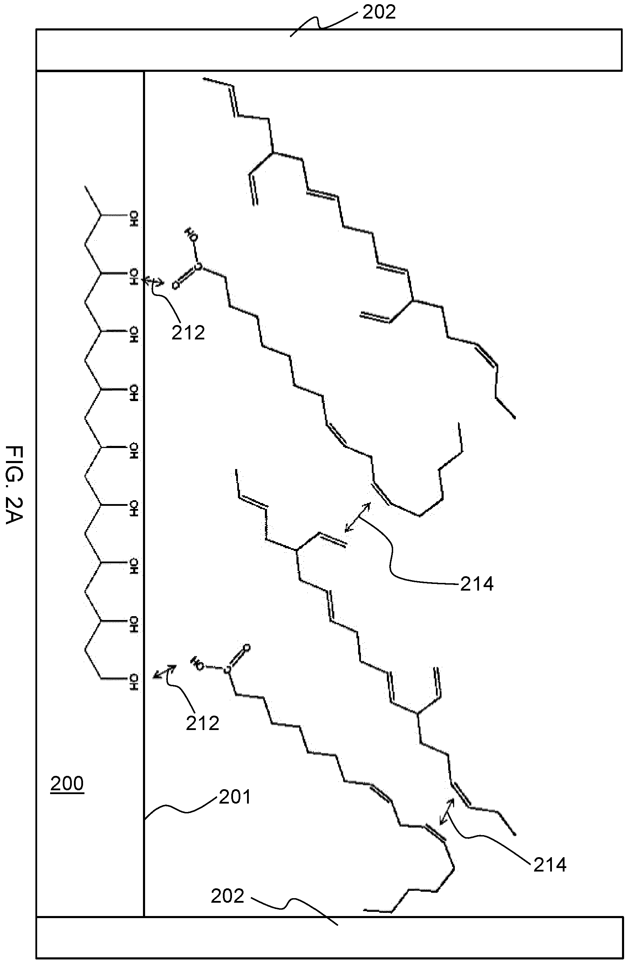

FIG. 2A schematically depicts, in a magnified side view which also shows the optional frame 202 and the surface 201 of the first polymer 200, orientating of the surfactant molecules. In this non-limiting example, the surfactant is linoleic acid, e.g. α-linolenic acid, and the orientating is such that a polar moiety thereof, in this case a carboxylic acid group, is made available at the second surface 207 of the polymer membrane 206, as previously described.

To this end a polar attraction, represented by the double-headed arrows 212, between the polar moiety of the surfactant and the first polymer, in this non-limiting example a polyvinylalcohol foil, assists to orientate the surfactant molecules such that the polar moiety is made available at the second surface 207 of the polymer membrane 206.

FIG. 2A thus shows how the polar moieties, in this case the carboxylic acid groups, are directed to, and interact with, the first polymer 200, in this case the first surface 201 of the polyvinyl alcohol foil, after dispensing of the solution 204.

In the non-limiting example schematically depicted in FIG. 2A, the second polymer has unsaturated carbon-carbon bonds, including vinyl groups, for crosslinking with unsaturated carbon-carbon bonds of the surfactant, as represented by the double-headed arrows 214.

In this specific example, such crosslinks are formed between the unsaturated carbon-carbon bonds, including vinyl groups, of the polybutadiene second polymer and the unsaturated carbon-carbon bonds of the linoleic acid surfactant.

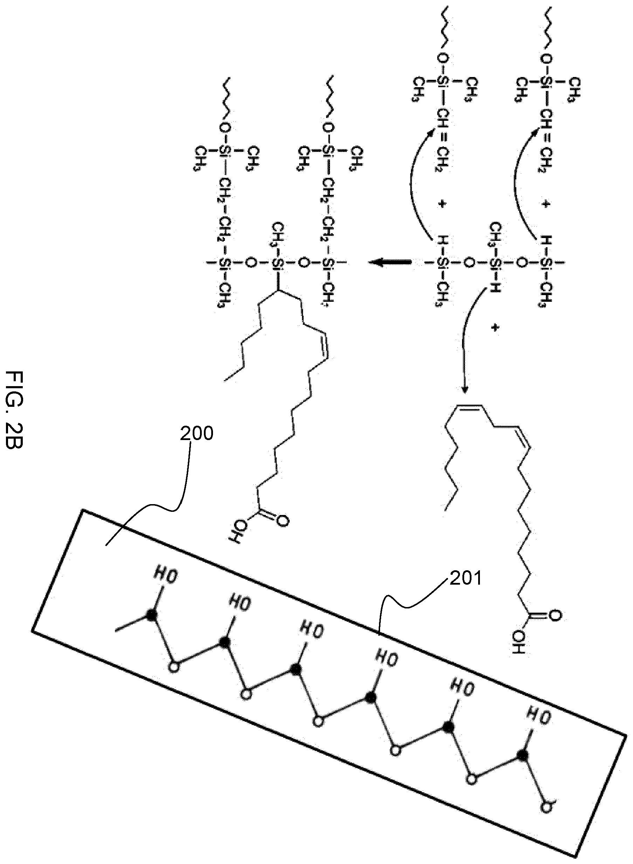

FIG. 2B shows an example in which crosslinks are formed via reaction between silicon-hydride bonds and the unsaturated carbon-carbon bonds of an unsaturated fatty acid surfactant, in this case linoleic acid. A similar reaction between silicon-hydride bonds and unsaturated carbon-carbon bonds takes place between the siloxane components shown in FIG. 2B.

A polar attraction between the polar moiety, in this case the carboxylic acid group, of the surfactant and the first polymer, in this non-limiting example a polyvinylalcohol foil, assists to orientate the surfactant molecules such that the polar moiety is made available at the second surface 207 of the polymer membrane 206, as previously described.

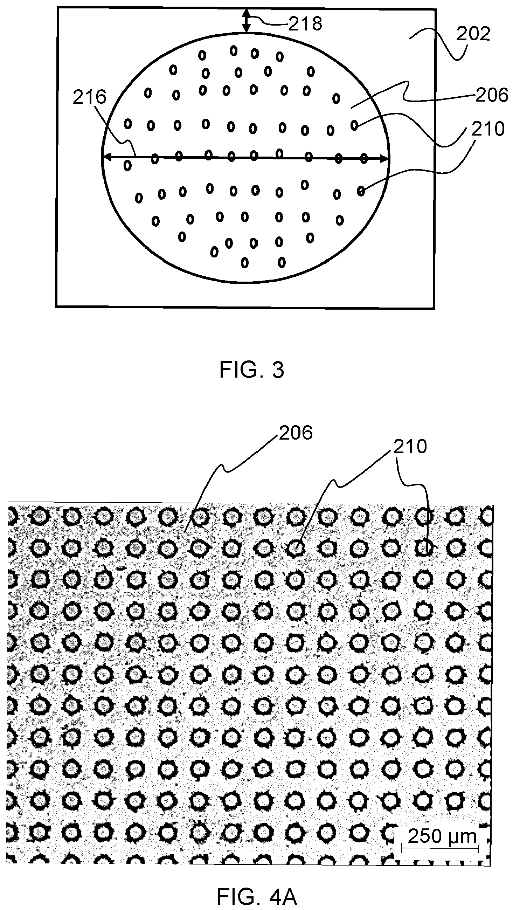

FIG. 3 provides a plan view of a polymer membrane 206 and a frame 202. In this non-limiting example, the polymer membrane 206 is positioned in a cavity delimited by the frame 202.

The frame 202 can be formed of any suitably stiff material, such as a metal or metal alloy, e.g. stainless steel, or a polymer, e.g. a polymer which is insoluble in the solvent(s) used in the above-described steps of the method 100. Examples of such a frame polymer are polyether ether ketone and a fluoropolymer, such as Teflon^{®}.

The dimensions and shape of the frame 202 can be selected according to the intended application, e.g. in a fluidic device, such as a cell culture device. In some embodiments, the frame 202 is also included, together with the polymer membrane(s) 206 in such a device.

The diameter 216 of the cavity can be, for example, in a range of 1-10 mm, such as 4-5 mm. The minimum width 218 of the frame 202 from the edge of the cavity to the outer edge of the frame 202 may be 1 mm.

The cavity can have any suitable shape, such as circular. Similarly any suitable overall frame shape can be considered. A rectangular/square frame 202 is shown in FIG. 3.

In the non-limiting example shown in FIG. 3, the diameter of the cavity is 5-6 mm, and the frame has an overall square shape measuring 10 mm by 10 mm.

Further evident in FIG. 3 are apertures 210 formed in the polymer membrane 206, e.g. via the above-mentioned laser drilling process.

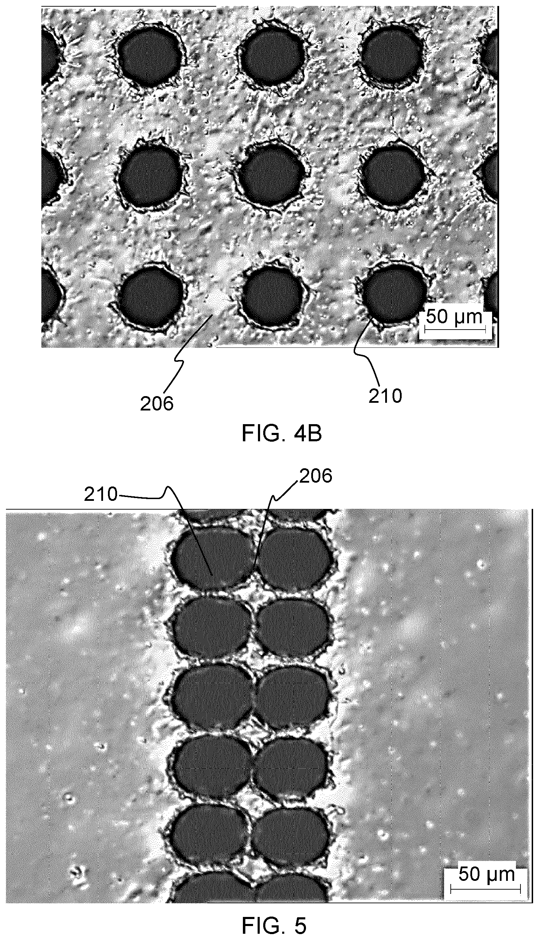

FIGs. 4A and 4B provide micrographs of part of an exemplary polymer membrane 206 having apertures 210 formed via laser drilling.

FIG. 5 provides a micrograph of part of another exemplary polymer membrane 206 having apertures 210 formed via laser drilling where minimum polymer membrane material is left between the apertures 210.

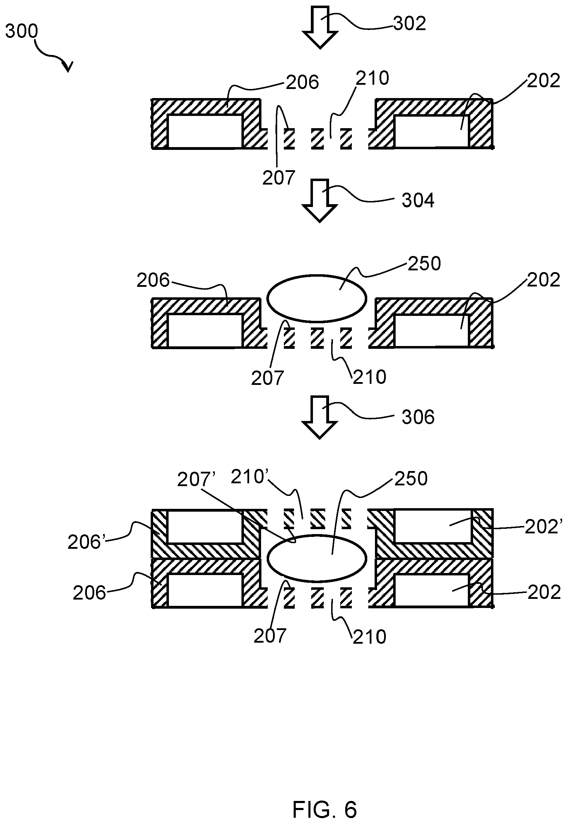

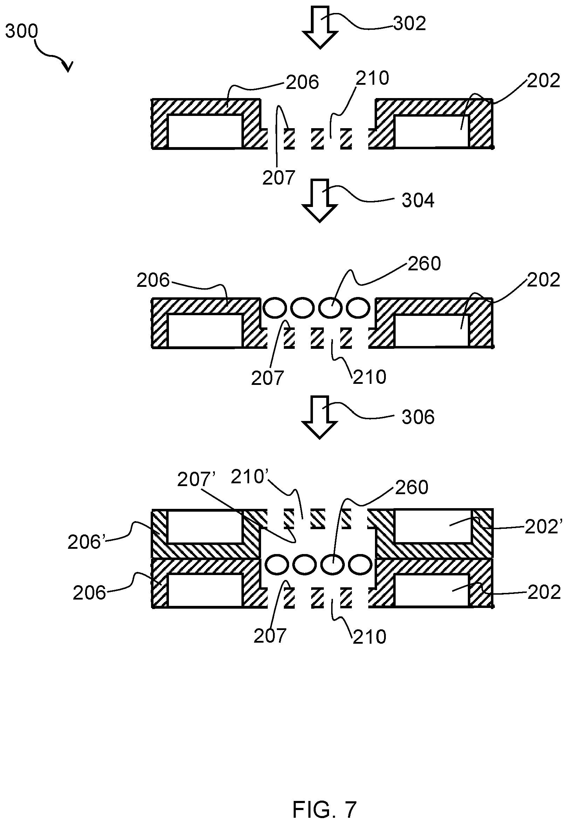

Further provided is a fluidic device comprising one or a plurality of the polymer membrane 206. FIG. 6 schematically depicts encapsulation of a tissue sample 250 by a plurality of polymer membranes 206, 206'. FIG. 7 schematically depicts encapsulation of spheroids/organoids 260 by a plurality of polymer membranes 206, 206'.

More generally a fluidic device, e.g. a cell culturing device, is provided which fluidic device comprises: a polymer membrane 206 and a further polymer membrane 206', the polymer membrane having a first surface 207 which opposes a second surface 207' of the further polymer membrane 206', at least one of the polymer membranes 206, 206' comprising a surfactant comprising surfactant molecules each having a polar moiety at the respective surface or surfaces.

One or both of the surfaces 207, 207' may be functionalized with a cell culturing protein. Such functionalizing may be via the polar moiety, e.g. via covalent bonding of the cell culturing protein to the polar moiety, as previously described.

One, or preferably both, of the polymer membranes 206, 206' may comprise one or more grooves and/or apertures 210, 210', as shown in FIGs. 6 and 7.

One or both of the polymer membranes 206, 206' may be fabricated, for example, according to any embodiment of the above-described method 100.

There is also provided a cell culturing method 300 comprising encapsulating cells with at least one of the polymer membrane 206 according to the present disclosure, for example between the above-described first and second polymer membranes 206, 206'.

Referring to FIG. 6, such a method 300 may comprise providing 302 a polymer membrane 206 comprising a surfactant comprising surfactant molecules each having a polar moiety at a surface 207 of the polymer membrane 206, the surface 207 being preferably functionalized with a cell culturing protein.

Cells may then be provided, e.g. dispensed, 304 on the surface 207 of the polymer membrane 206.

In the non-limiting example depicted in FIG. 6, the cells comprise or take the form of a tissue sample or biopsy 250. Alternatively, the cells comprise or take the form of spheroids/organoids 260, as shown in FIG. 7.

The method 300 further comprises providing 306 a further polymer membrane 206'. A further surface 207' of the further polymer membrane 206' opposes the polymer membrane 206, with the cells 250, 260 being thus encapsulated between the surfaces 207, 207' of the polymer membranes 206, 206'.

In some embodiments, the further polymer membrane 206' comprises a surfactant comprising surfactant molecules each having a polar moiety at the further surface 207', the further surface 207' being preferably functionalized with a cell culturing protein, e.g. such that both of the opposing surfaces 207, 207' are functionalized with a cell culturing protein.

One, or preferably both, of the polymer membranes 206, 206' may comprise one or more grooves and/or apertures 210, 210'. The groove(s) and/or aperture(s) 210, 210' may assist nutrients to be supplied and waste products to be removed from the cells via the groove(s) and/or aperture(s) 210, 210', and, in some examples, enable monitoring of the cell sample 250, 260, as previously described. Such aperture(s) 210 may, for example, have a diameter in the range of 1-1000 µm.

One of the polymer membranes 206, 206' can be regarded as a carrier membrane, and the other of the polymer membranes 206', 206 can be regarded as an encapsulation membrane. In such an example, neighboring apertures 210 of the encapsulation membrane 206 may be closer to each other than neighboring apertures 210' of the carrier membrane 206'. Alternatively or additionally, the apertures 210 of the encapsulation membrane 206 may have a larger diameter than the apertures 210' of the carrier membrane 206.

In many cases for cell growing, cell proliferation, cell growth to spheroids and organoids 260 and after placing a biopsy 250, it is undesirable for a cell to be able to pass through the polymer membrane 206. In this case, the sample 250, 260 may be placed on a polymer membrane 206 having apertures 210 with a diameter of, for example, 5-7 µm.

In examples in which it is desired to grow a double layer with epithelial cells on top of the polymer membrane 206 and endothelial cells on the other side of the polymer membrane 206, a key requirement is that the epithelial and endothelial cells do not become mixed up. Hence, the apertures 210 may be sufficiently small, e.g. 5-7 µm, to prevent cells from passing through the polymer membrane 206. In this way, the double layer of cells can grow and differentiate to an inner and an outer layer, e.g. to mimic a gut, lung or skin.

In examples in which it is desired only to hold spheroids and organoids 260 in place (e.g. with a geometry fixed for studying its growth and responses to a drug by using markers and staining) between two polymer membranes 206, 206', apertures 210, 210' can be provided in both polymer membranes 206, 206' smaller than the diameter of the spheroids/organoids.

In examples in which it is desired to fix the geometry of a biopsy 250, the polymer membrane 206 can be selected for cell permeability or non-cell permeability (depending on the needs, restrictions for the assay). The biopsy 250 can, for example, be kept in place in a moving fluid with a covering polymer membrane 206' on top with bigger apertures 210' which are more closely spaced to each other. A covering polymer membrane 206' with apertures 210' large enough to encapsulate, fix a biopsy 250 and relatively close to each other (in other words a relatively "open" polymer membrane 206') may be favorable for optical analysis via microscopy, e.g. due to such an open polymer membrane 206' facilitating focusing. Less material in the polymer membrane 206' may mean that more optical clearance is provided for imaging.

In some examples, polymer membranes 206 can be stacked, e.g. to bring more different organoids 260 together in one fluidic device, e.g. one organ-on-chip device. For instance, a liver organoid can stacked with a pancreas organoid between three membranes so that they can live/communicate/exchange proteins with each other.

In a further example, the metastasis of cancer cells coming from a cancer biopsy may be studied. In such an example, the diameter of the apertures 210 can be used to select whether or not cells are let through the polymer membrane 206 and thus filtered into an adjacent chamber where they can be collected, e.g. for imaging.

Hence, more generally, a fluidic device may comprise two polymer membranes 206, 206' whose apertures 210, 210' have different sizes relative to each other, with a first chamber defined between the polymer membranes 206, 206' and a second chamber separated from the first chamber by one of the polymer membranes 206, 206'.

It is further noted that the polymer membranes 206, 206' may be brought together using the frames 202, 202'. Thus, in such a non-limiting example, the frames 202, 202' may become parts of the fluidic device, e.g. cell culturing device, by which the polymer membranes 206, 206' are mounted.

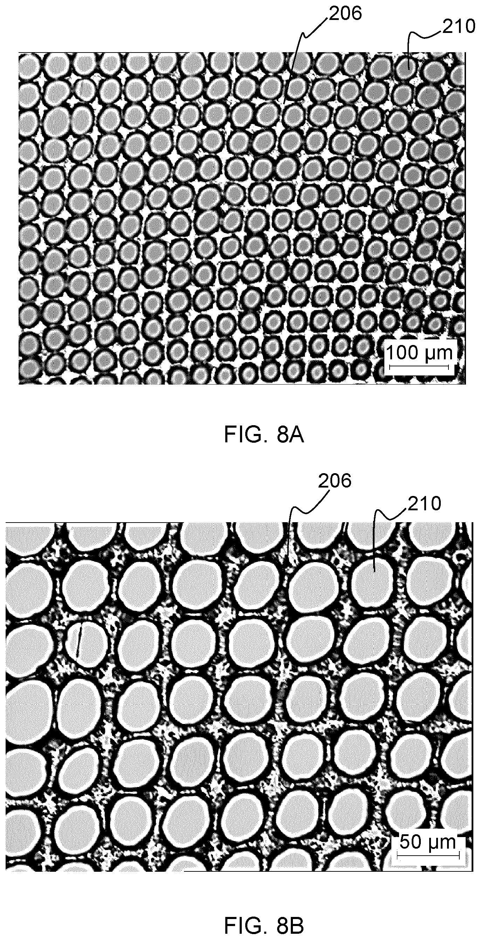

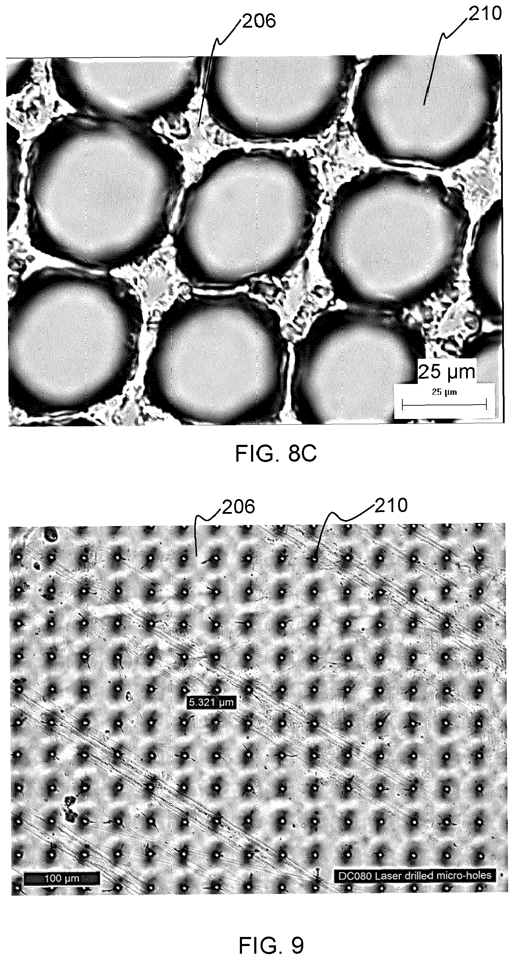

FIGs. 8A to 8C provide micrographs of an exemplary polymer membrane 206 for cell sample encapsulation, e.g. for use in the method 300 described above.

FIG. 9 provides a micrograph of an exemplary linoleic acid-modified polysiloxane polymer membrane 206, formed in the manner depicted in FIG. 2B, having laser-drilled apertures 210 each with a diameter of 5-7 µm. This polymer membrane206 is permeable for medium, proteins and drugs, but not for cells, since cell cores are 8 µm across.

A flexible soft membrane was fabricated by dispensing 104 a mixture of poly butadiene and unsaturated linoleic acid dissolved in heptane over a frame 202 onto a relatively polar polyvinylalcohol foil 200; carboxylic acid groups of the linoleic acid being directed to the surface 201 of the foil 200. After evaporating 106 the heptane and removal 110 of the foil, the resulting polymer membrane 206 was found to be straightforwardly mountable/incorporatable in a fluidic device, e.g. a "3D" fluidic device. The surface of the polymer membrane 206 has polar moieties, in this example carboxylic acid groups, where covalent bonds with proteins, e.g. human proteins, are possible. It is noted that such surface carboxylic acid groups can be detected using, for example, Oregon green 488 Cadaverin.

It is noted, in general, that the methods, polymer membranes and fluidic devices of the present disclosure are applicable in all kind of carriers for cell culturing and for any oncology tests in fluidic systems.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A method (100) of fabricating a polymer membrane suitable for a cell culture device, the method comprising:
providing (104) a solution (204) on a first surface (201) of a first polymer (200), the solution comprising a second polymer, a surfactant, and a solvent, wherein the surfactant comprises surfactant molecules each having a polar moiety; and
evaporating (106) the solvent while the solution is on the first surface to provide said polymer membrane (206), the polymer membrane having a second surface (207) in contact with the first surface, wherein at least said first surface of the first polymer has greater affinity for the polar moiety than the second polymer.

2. The method (100) according to claim 1, wherein the second polymer comprises an elastomer and/or has a Shore A hardness of 70 or less.

3. The method (100) according to claim 1 or claim 2, wherein the second polymer comprises at least one selected from a polydiene, a polysiloxane, and a polyurethane; optionally wherein the second polymer comprises polybutadiene.

4. The method (100) according to any of claims 1 to 3, wherein the first polymer (200) comprises one or more of polyvinylalcohol, polyurethane, and polyethylene glycol.

5. The method (100) according to any of claims 1 to 4, wherein the solvent comprises one or more of a hydrocarbon solvent, an alcohol solvent, and a ketone solvent.

6. The method (100) according to any of claims 1 to 5, wherein the polar moiety is a carboxylic acid group, an amine group or an amide group.

7. The method (100) according to any of claims 1 to 6, wherein the surfactant comprises a fatty acid, the carboxylic acid group of the fatty acid defining the polar moiety.

8. The method (100) according to any of claims 1 to 7, further comprising crosslinking the second polymer and at least some of the surfactant molecules; optionally wherein the second polymer comprises an unsaturated carbon-carbon bond and/or a hydride functional group, and said at least some of the surfactant molecules each comprise an unsaturated carbon-carbon bond, said crosslinking being via a reaction between the unsaturated carbon-carbon bond of the surfactant molecules and the unsaturated carbon-carbon bond and/or hydride functional group of the second polymer.

9. The method (100) according to any of claims 1 to 8, comprising separating (110) the polymer membrane (206) and the first polymer (200).

10. The method (100) according to claim 9, wherein said separating (110) comprises peeling the first polymer (200) and the polymer membrane (206) apart from each other and/or dissolving the first polymer in a solvent in which the polymer membrane is less soluble or insoluble so as to separate the polymer membrane from the first polymer.

11. The method (100) according to any of claims 1 to 10, comprising forming (114) one or more apertures and/or grooves (210) in the polymer membrane (206); optionally wherein the forming comprises laser drilling the polymer membrane.

12. The method (100) according to any of claims 1 to 11, comprising functionalizing the second surface (207) with a cell culturing protein; optionally wherein the cell culturing protein comprises fibronectin.

13. A polymer membrane (206) obtainable from the method according to any of claims 1 to 12.

14. A cell culturing device comprising one or a plurality of the polymer membrane (206) according to claim 13.

15. Acell culturing device comprising a polymer membrane (206) and a further polymer membrane (206'), the polymer membrane having a surface (207) which opposes a further surface (207') of the further polymer membrane, at least one of the polymer membranes comprising a surfactant comprising surfactant molecules each having a polar moiety at the respective surface or surfaces (207, 207').

16. A cell culturing method comprising immobilizing cells within a cell culturing device using at least one of said polymer membrane (206) according to claim 13.
